# EUROPEAN PATENT SPECIFICATION

(11) **EP 3 616 608 B1**
(45) Date of publication and mention of the grant of the patent: **14.06.2023**
(21) Application number: 19194673.0
(22) Date of filing: 30.08.2019
(51) Int. Cl.: A61B 5/259, A61B 5/282

(54) **PRINTED CONDUCTIVE LEADS FOR MEDICAL APPLICATIONS**
GEDRUCKTE LEITFÄHIGE LEITER FÜR MEDIZINISCHE ANWENDUNGEN
FILS CONDUCTEURS IMPRIMÉS POUR APPLICATIONS MÉDICALES

(30) Priority: 03.09.2018 US 201816120358
(43) Date of publication of application: 04.03.2020
(73) Proprietor: Nikomed USA, Inc., Hatboro, Pennsylvania 19040 (US)
(72) Inventor: EPSTEIN, Stephen T., Newtown, Pennsylvania 18940 (US)
(74) Representative: MacLachlan & Donaldson

(56) References cited:
- US-A- 5 341 806
- US-A1- 2010 076 295
- US-A1- 2011 054 286

## Description

### BACKGROUND OF THE INVENTION

### 1. Field Of The Invention

In general, the present invention relates to the structure of conductive lead elements that interconnect probes and sensors to medical analysis equipment.

### 2. Prior Art Description

Many medical testing and monitoring systems require that various probes and sensors be attached to the body of a patient. For example, to monitor blood oxygen levels, a pulse oximeter is commonly applied to the tip of the finger. To perform an electrocardiogram, multiple sensors are attached to the torso and limbs. In each case, the sensor or probe is attached to medical equipment using wire leads. This presents multiple problems.

The primary problem with the wire leads is one of expense. Traditional wire leads contain copper wire, which is expensive. Furthermore, many wire leads have complex structures. For instance, the wire leads that interconnect with a pulse oximeter contain two sets of copper wires that are each shrouded in a conductive sheath to prevent signal interference. The complexity of the wire lead adds significantly to its cost. Many hospitals routinely use wire leads for only one patient and throw the wire leads away each time a patient is discharged. The replacement costs associated with replacing wire leads costs hospitals, clinics and physicians' offices is many millions of dollars each year.

The wire leads for medical equipment, such as electrocardiograms, are so extensive and complex that they are rarely replaced. Rather, many hospitals, clinics and physicians' offices use disposable probes and repeatedly connect the probes using the same wiring harnesses. This, of course, presents problems with patient-to-patient contamination. Wiring harnesses come into contact with a patient's skin and clothing. As such, they can be contaminated with bacteria, viruses, blood and/or other bodily fluids. As a consequence, healthcare providers are required to balance the risks and costs associated with replacing or reusing wire leads. Healthcare providers must either absorb the large expense of replacing or sterilizing wire leads after each use, or they must assume the dangers and complications of potentially cross-contaminating patients by reusing wire leads.

Another disadvantage of traditional wire leads is that the connectors at the ends of the leads wear over time. As the connectors wear, they often become loose and create weak electrical connections with the probe or sensor to which it is attached.

In the prior art, attempts have been made to replace expensive wire leads with less expensive elements, such as printed flexible substrates. Such prior art is exemplified by U.S. Patent No. 6,006,125 to Kelly and U.S. Patent Application Publication No. 2004/0210149 to Wenger, both of which are used for electrocardiograms. The problems associated with such printed substrates, is that they are printed in one size in the hope that one size fits all people. This is clearly not accurate. An infant is obviously very different in size than a full-grown adult. Wire leads can be manipulated to accommodate people of different sizes. However, printed leads are fixed on a substrate. As such, the premanufactured printed leads should be produced in a wide variety of sizes and styles to accommodate people of different ages, sizes, shapes and genders. This requires preprinted substrates of many different sizes and lengths to be held in the inventory of a hospital or clinic. The consequence is that large sums of money must be spent on inventory. This negates the cost savings of not using traditional lead wires.

US 5 341 806 discloses an electrode strip for use in electrocardiography comprising a flexible and substantially inextendible substrate, a plurality of conductors that extend along the substrate to form a plurality of electrode sites (V₁ - V₆, RA, LA, LL, RL), and a cover layer that insulates the conductors. A plurality of regions of extensibility in the strip allow selective positioning of the electrode sites on a body.

US 2011/054286 discloses an expandable electrode pad having a flexible and stretchable base member that may be either expanded or compressed to provide proper positioning for a plurality of electrode distal contacts. Portions of the base member may have perforations that allow one or more sections of the base member to be separated and positioned a greater, non-stretchable distance away from the remaining strip of distal contacts. Additionally, the circuit body may comprise a distal cut zone that provides for freedom of movement of the plurality of distal contacts and a proximal perforated zone that eliminates lead wire entanglements while also allowing for the tearing of perforations between adjacent conductive circuits when a greater reach is required for the proper placement of a distal contact.

US 2010/076295 discloses an electrode set which includes a base comprising an insulating material, and a plurality of electrodes disposed on the base. The electrodes comprise a generally exposed conductive material. The electrode set also includes a plurality of conductors disposed on the base. The conductors comprise a generally insulated conductive material, and are each connected to one of the electrodes. The electrodes are positioned on the base relative to each other so that they can be collectively placed on specific portions of a patient's anatomy.

A need therefore exists for new lead elements for medical equipment that can be universally used on all patients, regardless of size, shape, age or gender. A need also exists for such lead elements that are highly reliable, yet inexpensive enough to be replaced after every use. Lastly, a need exists for new lead elements that can be manufactured at a price that is far less expensive than the cost of traditional wire leads or the cost of sanitizing traditional wire leads. These needs are met by the present invention as described and claimed below.

### SUMMARY OF THE INVENTION

The present invention is a flexible connector system for connecting a patient to a piece of medical monitoring equipment. The flexible connector system includes a sensor for detecting biomedical signals and a ribbon connector that connects the sensor to the medical monitoring equipment.

The ribbon connector includes a plurality of conductive leads that are printed upon a dielectric flexible substrate in a conductive ink. The ribbon connector has a first length between a first end and an opposite second end. The ribbon connector has a first section proximate the first end and a second section proximate the second end. The second section is perforated in a manner that enables that section of the ribbon connector to be separated into strips. Once separated, each of the strips supports a separate conductive lead.

Contact pads are printed on the flexible substrate proximate the first end and the second end of said ribbon connector. The contact pads terminate the various conductive leads. A first insulation layer covers a plurality of conductive leads between the contact pads to prevent incidental shorting of the conductive leads. The first insulation layer can be covered with a conductive shielding layer and a second insulation layer to prevent electro-magnetic interference with signals passing through the conductive leads.

### BRIEF DESCRIPTION OF THE DRAWINGS

For a better understanding of the present invention, reference is made to the following description of exemplary embodiments thereof, considered in conjunction with the accompanying drawings, in which:
FIG. 1 shows the present invention system being used to connect a patient to a piece of medical equipment;
FIG. 2 is a top view of a ribbon connector with connected perforated strips;
FIG. 3 is a top view of a ribbon connector with separated perforated strips;
FIG. 4 is a cross-sectional view of the ribbon connector shown in Fig. 2;
FIG. 5 is a top view of a ribbon connector with a conductive sheath;
FIG. 6 is a cross-sectional view of the ribbon connector shown in Fig. 5;
FIG. 7 is a perspective view of an exemplary sensor engaging a strip of the ribbon connector;
FIG. 8 is a top view of the assembled embodiment of Fig. 7;
FIG. 9 shows a perspective view of an exemplary sensor connector termination for the ribbon connector that joins the ribbon connector to a standard medical sensor;
FIG. 10 shows an exploded view of the sensor connector shown in Fig. 9; and
FIG. 11 shows a cross-sectional view of the sensor connector shown in Fig. 9.

### DETAILED DESCRIPTION OF THE DRAWINGS

Although the present invention system and method can be embodied in many ways, only two exemplary embodiments are shown. These embodiments are selected in order to set forth some of the best modes contemplated for the invention. The illustrated embodiments, however, are merely exemplary and should not be considered limitations when interpreting the scope of the claims.

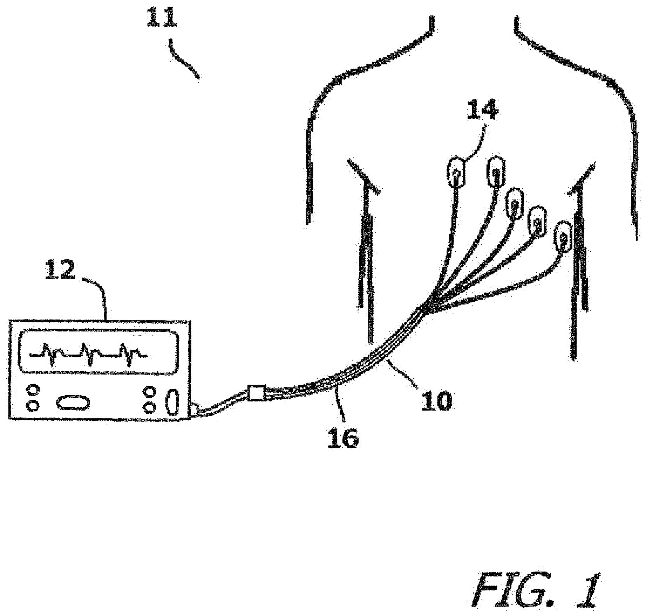

Referring to Fig. 1, a system 11 is shown where a ribbon connector 10 is used to interconnect a piece of medical equipment 12 to sensors 14 on a patient's body. The ribbon connector 10 contains no metal wires. Rather, as is later explained, the ribbon connector 10 contains parallel conductive leads 16 that are printed upon the ribbon connector 10. In the shown embodiment, the ribbon connector 10 and its component conductive leads 16 are being used in an electrocardiogram (ECG), wherein the sensors 14 are ECG pads and are placed on the body in a variety of positions. The placement of the sensors 14 is dependent upon the size and anatomy of the person being monitored.

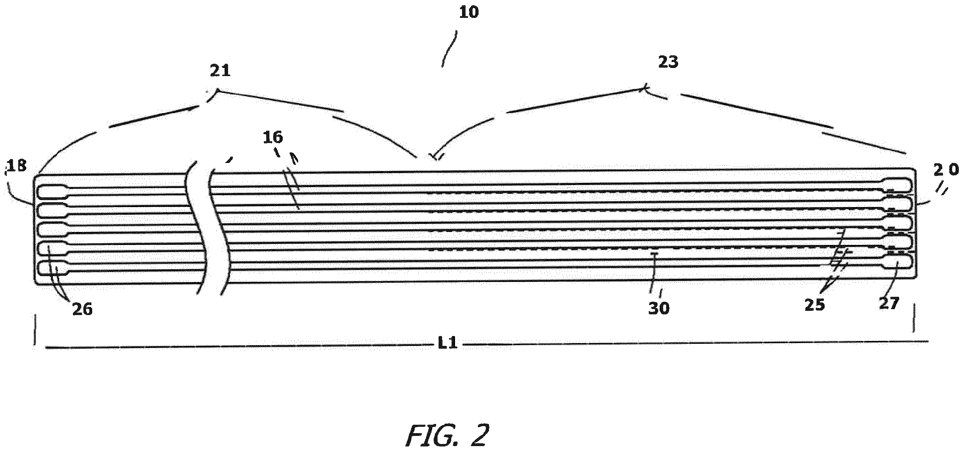

Referring to Fig. 2 in conjunction with Fig. 1, it will be understood that the ribbon connector 10 has a first end 18 that attaches to the piece of medical equipment 12 and a second end 20 that attaches to the medical sensors 14 on the patient's body. The purpose of the ribbon connector 10 is to conduct electrical signals between the medical sensors 14 and the piece of medical equipment 12. Between the first end 18 and the second end 20, the ribbon connector 10 is divided into two sections. The first section 21 extends from the first end 18 toward the second end 20. The first section 21 is solid. That is, the ribbon connector 10 is a single piece. The second section 23 extends from the first section 21 to the section end 20. In the second section 23, the ribbon connector 10 is perforated so it can be divided into strips 25.

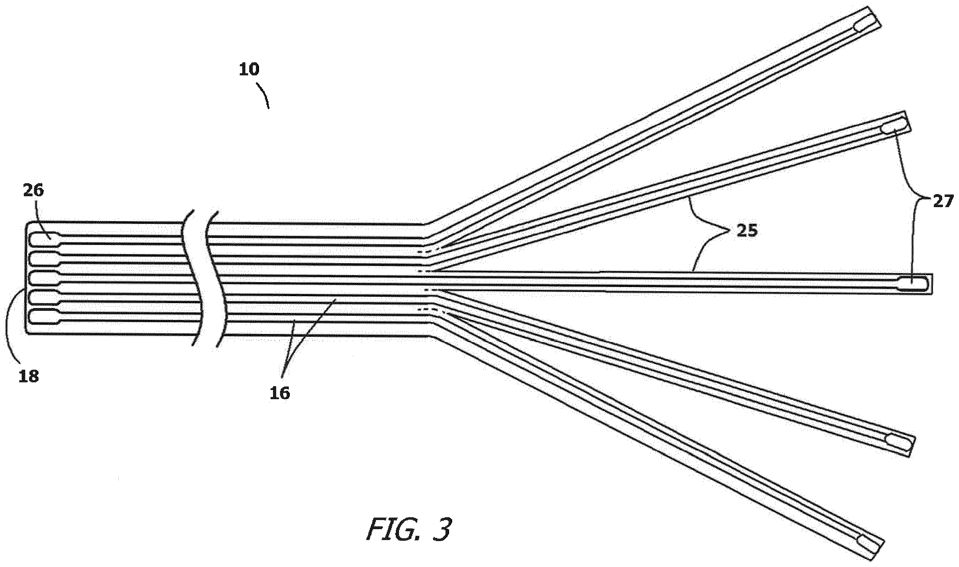

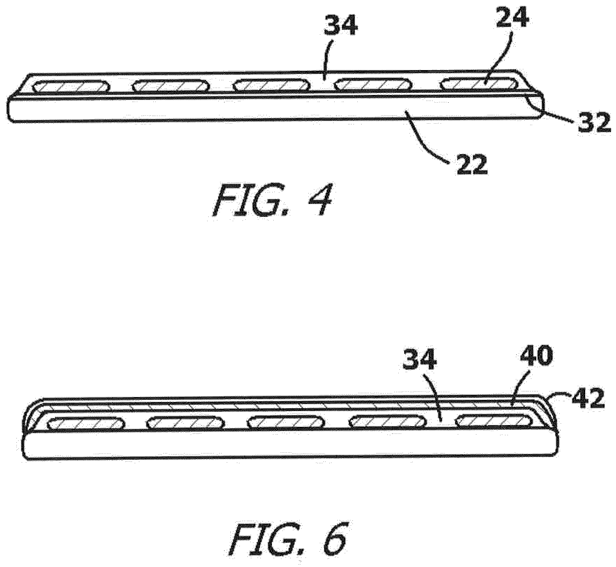

Referring to Fig. 2 in conjunction with Fig. 3 and Fig. 4, it can be seen that the ribbon connector 10 has a length L1, which is preferably between eight inches and thirty-six inches. The ribbon connector 10 is manufactured upon a thin substrate 22 that has a preferred thickness of between 5 mil and 20 mils, so as to be highly flexible. The preferred substrate 22 is a film of polyethylene terephthalate (PET). However, films of flashspun non-woven high-density polyethylene fiber, such as Tyvek^{®}, can also be used. These films are dielectric, highly flexible, and tear-resist in tension.

A conductive ink 24 is printed upon the flexible substrate 22 to form each conductive lead 16. In the shown embodiments, there are either five or six parallel conductive leads 16 shown. However, it will be understood that any plurality of conductive leads 16 can be formed, depending upon the needs of the piece of medical equipment 12 being utilized. Each conductive lead 16 extends from the first end 18 of the ribbon connector 10 to the second end 20 of the ribbon connector 10. Contact pads 26 are formed on each conductive lead 16 near the first end 18 of the ribbon connector 10 to enable the conductive leads 16 to electrically interconnect with the medical equipment 12. Likewise, contact pads 27 are formed on each conductive lead 16 near the second end 20 of the ribbon connector 10 to facilitate the connection of the contact leads to various probes and sensors.

Each conductive lead 16 is electrically isolated from the other conductive leads 16 in the same ribbon connector 10. Furthermore, the ribbon connector 10 is perforated in the second section 23 approaching the second end 20 of the ribbon connector 10. Depending upon the length of the ribbon connector 10, the second section 23 can be up to half as long as the overall length L1 of the ribbon connector 10. The perforations 30 in the perforated area 28 divide the ribbon connector into strips 25. Each of the strips 25 supports a different one of the conductive leads 16. This enables each of the conductive leads 16 to be oriented in different directions to reach the various sensors 14 that are positioned on a patient's body.

The conductive leads 16 are deposits of conductive ink 24 that are printed upon the material of the substrate 22. The conductive ink 24 is preferably applied using an industrial grade electronic printer. However, alternate printing methods, such as silk-screening, can also be used. Many conductive inks can be used in the printing. However, to limit distortion and cracking of the ink, silver-based inks are preferred. The preferred silver-based ink is an Ag/AgCI ink. An Ag/AgCI ink transfers an electrical charge across its boundaries by a reversible redox reaction. Accordingly, an Ag/AgCI ink is not polarizable and will not filter or otherwise alter the electrical signal from a medical sensor 14.

Depending upon the composition of the flexible substrate 22 and the composition of the conductive ink 24, the flexible substrate 22 may be coated with an aqueous primer 32 that increases the adhesion between the conductive ink 24 and the flexible substrate 22. There are many primers commercially available that are used to print ink onto PET or high-density polyethylene fiber. Many of these primers work with silver-based conductive inks and can be incorporated into the present invention. The primer 32 prevents the conductive ink 24 from peeling away from the flexible substrate 22 if the flexible substrate 22 is severely deformed during processing and/or use. The primer 32 is also beneficial in the adhesion of any insulation layer over the flexible substrate 22 and conductive ink 24.

A first dielectric insulation layer 34 is applied over both the conductive ink 24 and the flexible substrate 22. The first dielectric insulation layer 34 is a layer of dielectric ink that is printed over the conductive ink 24 and the exposed flexible substrate 22. The dielectric ink encapsulates the conductive ink 24 so that the conductive ink 24 is interposed between the flexible substrate 22 and the dielectric ink. Alternatively, not being part of the invention, the first dielectric insulation layer 34 can be a curable dielectric polymer that is sprayed or otherwise mechanically applied over the conductive ink 24 and the exposed flexible substrate 22. In either manufacturing scenario, the conductive ink 24 is interposed between the flexible substrate 22 and the first dielectric insulation layer 34. Accordingly, the conductive ink 24 cannot short against the skin or any metallic object that it may inadvertently contact.

As is shown in Fig. 2 and Fig. 3, the various conductive leads 16 extend from the first end 18 to the second end 20 of the ribbon connector 10. Enlarged contact pads 26, 27 terminate both ends of the conductive leads 16 proximate the first end 18 and the second end 20 of the ribbon connector 10. The enlarged contact pads 26, 27 are large areas of conductive ink 24 that are printed in the same manner as the conductive leads 16 are printed.

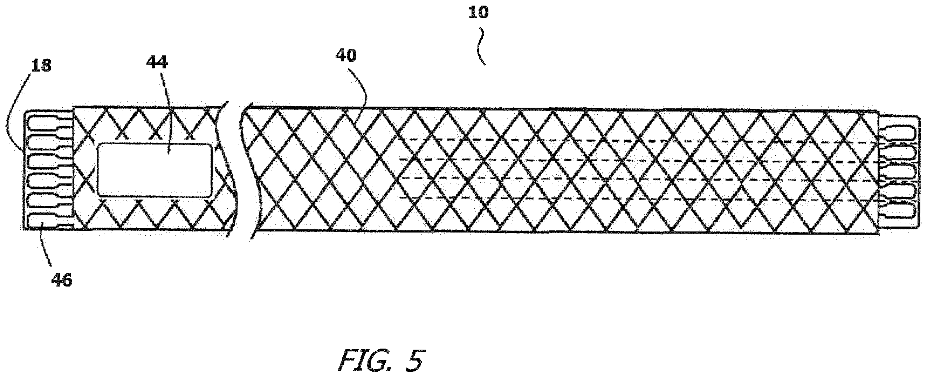

Referring to Fig. 5 and Fig. 6, it can be seen that additional layers can be added to the ribbon connector 10 to shield the ribbon connector 10 from electro-magnetic interference. A shielding layer 40 is printed upon the first insulation layer 34. The shielding layer 40 is made from conductive ink. The conductive ink can be inexpensive, such as an aluminum alloy ink, and need not be silver-based. The shielding layer 40 is preferably not solid, but is rather printed in some mesh pattern that minimizes the amount of conductive material used.

The shielding layer 40 is covered in a second insulation layer 42 to prevent inadvertent contact of the shielding layer 40. A field 44 of white ink or the like can be printed atop the second insulation area. This field 44 can be used to adhere and/or write patient information. In this manner, a ribbon connector 10 can be used on a particular patient and will not be used on any other patient where it can cause cross contamination.

If a shielding layer 40 is utilized, the shielding layer 40 is electrically connected to an extra contact pad 46 at the first end of the ribbon connector 10. In this manner, the extra contact pad 46 will be connected to ground when the ribbon connector 10 is attached to a piece of medical equipment. The extra contact pad 46 used for grounding the shielding layer 40 is only present at the first end 18 of the ribbon connector 10. As such, the first end 18 of the ribbon connector 10 has one more contact pad than does the second end 20 of the ribbon connector 10.

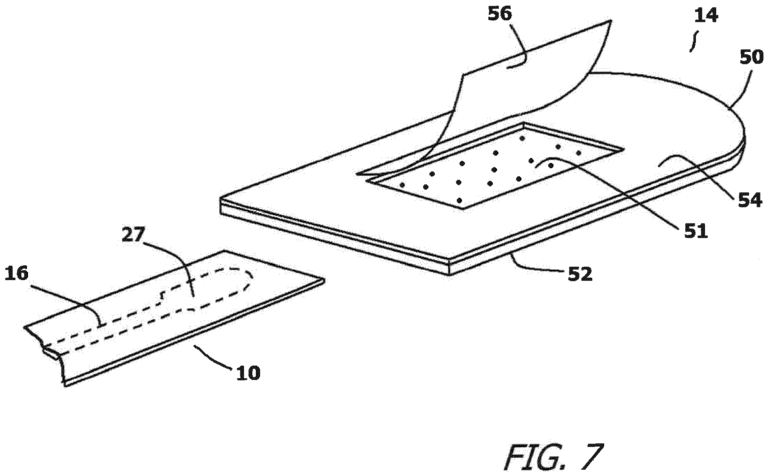

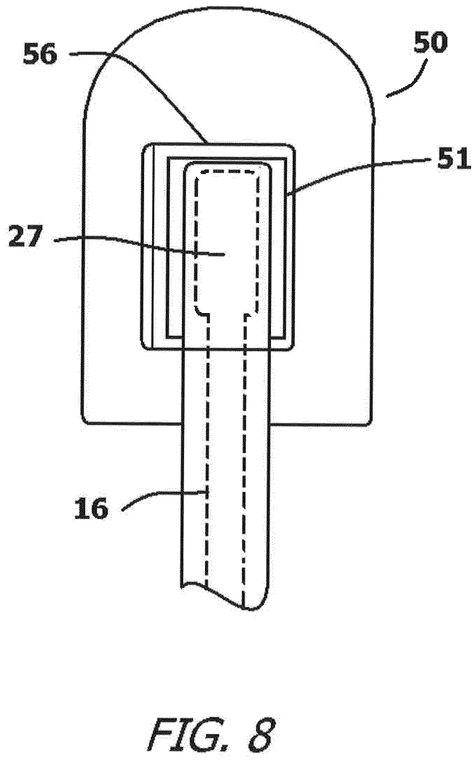

Referring to Fig. 7 in conjunction with Fig. 8, Fig. 2 and Fig. 3, it will be understood that the purpose of the ribbon connector 10 is to electrically interconnect a piece of medical equipment to a probe or sensor 14. In the shown illustration, the sensor 14 is a specialized ECG sensor pad 50 that is adapted for use as part of the present invention system. The specialized ECG sensor pad 50 has a conductive layer 52 that adheres to the skin of a patient. The conductive layer 52 is supported by a dielectric substrate 54. A window 51 is formed in the dielectric substrate 54 that provides access to the conductive layer 52. The window 51 is covered by an adhesive flap 56.

To connect one of the conductive leads 16 from the ribbon connector 10 to the sensor 14, the adhesive flap 56 is pulled open. The contact pad 27 on the second end of the conductive lead 16 is then placed in flush contact against the sensor's conductive layer 52 within the window 51. The adhesive flap 56 is closed over the conductive lead 16, therein locking the conductive lead 16 in place. Electrical contact is made between the contact pad 27 at the end of the conductive lead 16 and the conductive layer 52 of the sensor 50.

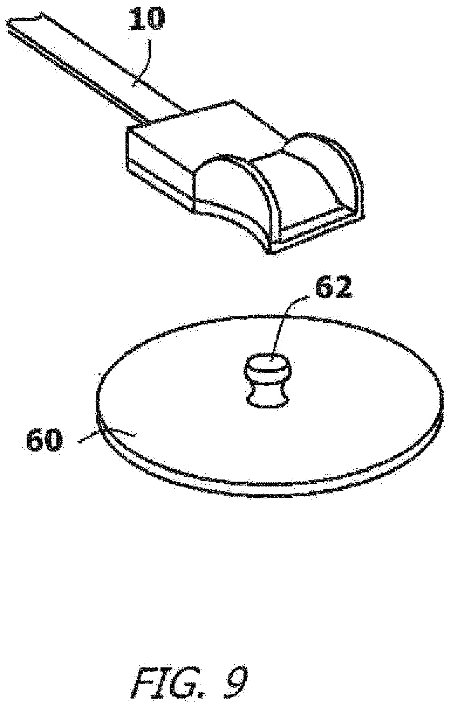

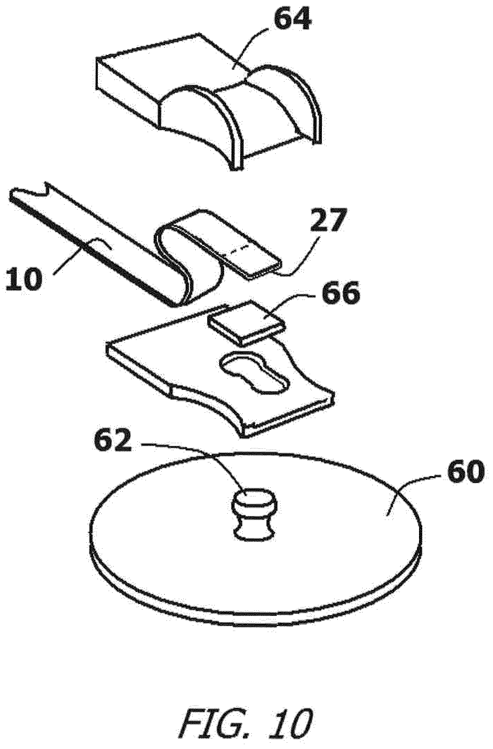

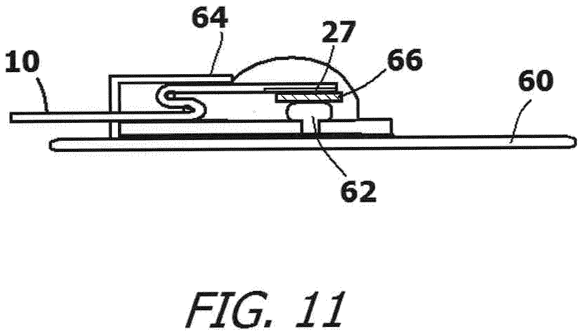

The embodiment of Fig. 7 and Fig. 8 show a specialized ECG sensor pad 50 that is adapted for use with the ribbon connector 10. However, it should be understood that the ribbon connector 10 can also be attached to standard sensor designs. Referring to Fig. 9, Fig. 10 and Fig. 11, an alternate sensor connection is illustrated, wherein the ribbon connector 10 is connected to a standard ECG sensor 60. A standard ECG sensor 60 has a central conductive post 62. A terminating connector 64 is provided that joins the ribbon connector 10 to the conductive post 62.

The terminating connector 64 positions the conductive pad 27 at the second end of the ribbon connector 10 in contact with a conductive contact plate 66. The terminating connector 64 mechanically engages the conductive post 62 and biases the contact plate 66 against the conductive post 62. This creates conductivity from the conductive post 62 to the ribbon connector 10. The ribbon connector 10 passes through a serpentine pathway in the terminating connector 64 to secure the ribbon connector 10 in place and to prevent the rib bon connector 10 from being pulled out of the connector 64.

It will be understood that the embodiments of the present invention that are illustrated and described are merely exemplary and that a person skilled in the art can make many variations to those embodiments. For example, the sensor can be an ECG sensor, a blood oxygen sensor or any other medical sensor that is traditionally attached to the body and has a wire lead. Likewise, the ribbon connector can be manufactured for use with different sensors, wherein the ribbon connector divides into as many leads as are necessary to connect to the sensors on the body. All such embodiments are intended to be included within the scope of the present invention as defined by the appended claims.

## Claims

1. A flexible connector system (11) for connecting a patient to a piece of medical monitoring equipment, said flexible connector system (11) comprising: at least one sensor (14) configured to be positioned on the patient's body to detect biomedical signals;
a flexible substrate (22) of dielectric material;
a plurality of conductive leads (16) printed in a conductive ink upon said flexible substrate (22), therein forming a ribbon connector (10), which is configured to electrically interconnect the piece of medical monitoring equipment (12) to the at least one sensor (14),
wherein said ribbon connector (10) has a first length between a first end (18) and an opposite second end (20), and wherein said ribbon connector (10) has a first section (21) that extends toward said second end (20) from said first end (18);
a plurality of perforations (30) formed in said flexible substrate (22) in a second section (23) that extends from said first section (21) to said second end (20), wherein said perforations (30) divide said flexible substrate (22) into a plurality of strips (25), and wherein each of said strips (25) supports one of said plurality of conductive leads (16);
contact pads (26, 27) printed on said flexible substrate (22) proximate said first end (18) and said second end (20) of said ribbon connector (10), wherein said contact pads (26, 27) terminate said plurality of conductive leads (16); and
a first insulation layer (34) printed in a dielectric ink over said plurality of conductive leads (16) between said contact pads (26, 27), wherein said dielectric ink encapsulates said conductive ink so that said conductive ink is interposed between said dielectric ink and said dielectric material of said flexible substrate (22);
a conductive shielding layer (40) made from conductive ink printed over said first insulation layer (34); and
a second insulation layer (42) which covers said conductive shielding layer (40).

2. The system according to Claim 1, further including sensors (14) affixed to each of said strips (25), wherein said plurality of conductive leads (16) are configured to electrically interconnect with said sensors (14).

3. The system according to Claim 1 or Claim 2, further including a ground contact pad (46) printed at said first end (18) of said ribbon conductor (10), wherein said conductive shielding layer (40) is electrically interconnected with said ground contact pad (46).

4. The system according to any preceding Claim, wherein said conductive shielding layer (40) is printed on said first insulation layer (34) in a mesh pattern.

5. The system according to Claim 1, wherein said second section (23) has a length up to half said first length of said ribbon connector (10).

6. The system according to any preceding claim wherein said first section (21) is solid.

7. The system according to any preceding claim comprising:
a plurality of sensors;
wherein each of said strips (25) terminates with one of said plurality of sensors (14).

8. The system according to Claim 7, wherein said plurality of strips (25) of said ribbon connector (10) are adhesively affixed to said plurality of sensors (14).

## Patentansprüche

1. Flexibles Verbindersystem (11) zum Verbinden eines Patienten mit einem Teil eines medizinischen Überwachungsgeräts, das flexible Verbindersystem (11) umfassend:
mindestens einen Sensor (14), der konfiguriert ist, um an dem Körper des Patienten positioniert zu werden, um biomedizinische Signale zu erfassen;
ein flexibles Substrat (22) aus dielektrischem Material;
eine Vielzahl von leitenden Leitungen (16), die mit einer leitenden Tinte auf das flexible Substrat (22) gedruckt sind und darin einen Bandverbinder (10) bilden, der konfiguriert ist, um das Teil eines medizinischen Überwachungsgeräts (12) elektrisch mit dem mindestens einen Sensor (14) zu verbinden,
wobei der Bandverbinder (10) eine erste Länge zwischen einem ersten Ende (18) und einem gegenüberliegenden zweiten Ende (20) aufweist, und wobei der Bandverbinder (10) einen ersten Abschnitt (21) aufweist, der sich von dem ersten Ende (18) zu dem zweiten Ende (20) erstreckt,
eine Vielzahl von Perforationen (30), die in dem flexiblen Substrat (22) in einem zweiten Abschnitt (23) gebildet sind, der sich von dem ersten Abschnitt (21) zu dem zweiten Ende (20) erstreckt, wobei die Perforationen (30) das flexible Substrat (22) in eine Vielzahl von Streifen (25) unterteilen, und wobei jeder der Streifen (25) eine der Vielzahl von leitenden Leitungen (16) trägt;
Kontaktflächen (26, 27), die in der Nähe des ersten Endes (18) und des zweiten Endes (20) des Bandverbinders (10) auf das flexible Substrat (22) gedruckt sind, wobei die Kontaktflächen (26, 27) die Vielzahl von leitenden Leitungen (16) abschließen; und
eine erste Isolierschicht (34), die mit einer dielektrischen Tinte zwischen den Kontaktflächen (26, 27) über die Vielzahl von leitenden Leitungen (16) gedruckt ist, wobei die dielektrische Tinte die leitende Tinte einkapselt, sodass die leitende Tinte zwischen der dielektrischen Tinte und dem dielektrischen Material des flexiblen Substrats (22) eingefügt ist,
eine leitende Abschirmschicht (40) aus leitender Tinte, die über die erste Isolierschicht (34) gedruckt ist; und
eine zweite Isolierschicht (42), die die leitende Abschirmschicht (40) bedeckt.

2. System nach Anspruch 1, das ferner Sensoren (14) beinhaltet, die an jedem der Streifen (25) befestigt sind, wobei die mehreren leitenden Leitungen (16) konfiguriert sind, um mit den Sensoren (14) elektrisch verbunden zu sein.

3. System nach Anspruch 1 oder Anspruch 2, das ferner eine Erdungskontaktfläche (46) beinhaltet, die auf das erste Ende (18) des Bandleiter (10) gedruckt ist, wobei die leitende Abschirmschicht (40) elektrisch mit der Erdungskontaktfläche (46) verbunden ist.

4. System nach einem vorherigen Anspruch, wobei die leitende Abschirmschicht (40) in einem Netzmuster auf die erste Isolierschicht (34) gedruckt ist.

5. System nach Anspruch 1, wobei der zweite Abschnitt (23) eine Länge bis zu der Hälfte der ersten Länge des Bandverbinders (10) aufweist.

6. System nach einem vorherigen Anspruch, wobei der erste Abschnitt (21) massiv ist.

7. System nach einem vorherigen Anspruch, umfassend:
eine Vielzahl von Sensoren;
wobei jeder der Streifen (25) mit einem der Vielzahl von Sensoren (14) abgeschlossen wird.

8. System nach Anspruch 7, wobei die Vielzahl von Streifen (25) des Bandverbinders (10) klebend an der Vielzahl von Sensoren (14) befestigt sind.

## Revendications

1. Système de connexion souple (11) permettant de connecter un patient à un élément d'équipement de surveillance médicale, ledit système de connexion souple (11) comprenant :
au moins un capteur (14) conçu pour être positionné sur le corps du patient afin de détecter des signaux biomédicaux ;
un substrat souple (22) en matériau diélectrique ;
une pluralité de fils conducteurs (16) imprimés dans une encre conductrice sur ledit substrat souple (22), formant en son sein un connecteur ruban (10), qui est conçu pour interconnecter électriquement l'élément d'équipement de surveillance médicale (12) à au moins un capteur (14),
ledit connecteur ruban (10) présentant une première longueur entre une première extrémité (18) et une seconde extrémité opposée (20), et ledit connecteur ruban (10) présentant une première section (21) qui s'étend en direction de ladite seconde extrémité (20) depuis ladite première extrémité (18) ;
une pluralité de perforations (30) formées dans ledit substrat souple (22) dans une seconde section (23) qui s'étend de ladite première section (21) à ladite seconde extrémité (20), lesdites perforations (30) divisant ledit substrat souple (22) en une pluralité de bandes (25), et chacune desdites bandes (25) supportant l'un de ladite pluralité de fils conducteurs (16) ;
des plots de contact (26, 27) imprimés sur ledit substrat souple (22) à proximité de ladite première extrémité (18) et de ladite seconde extrémité (20) dudit connecteur ruban (10), lesdits plots de contact (26, 27) terminant ladite pluralité de fils conducteurs (16) ; et
une première couche isolante (34) imprimée dans une encre diélectrique sur ladite pluralité de fils conducteurs (16) entre lesdits plots de contact (26, 27), ladite encre diélectrique encapsulant ladite encre conductrice de sorte que ladite encre conductrice soit interposée entre ladite encre diélectrique et ledit matériau diélectrique dudit substrat souple (22) ;
une couche de blindage conductrice (40) constituée d'une encre conductrice imprimée sur ladite première couche isolante (34) ; et
une seconde couche isolante (42) qui recouvre ladite couche de blindage conductrice (40).

2. Système selon la revendication 1, comprenant en outre des capteurs (14) fixés à chacune desdites bandes (25), ladite pluralité de fils conducteurs (16) étant conçus pour s'interconnecter électriquement avec lesdits capteurs (14).

3. Système selon la revendication 1 ou la revendication 2, comprenant en outre un plot de contact de masse (46) imprimé au niveau de ladite première extrémité (18) dudit conducteur ruban (10), ladite couche de blindage conductrice (40) étant électriquement interconnectée avec ledit plot de contact de masse (46).

4. Système selon l'une quelconque des revendications précédentes, ladite couche de blindage conductrice (40) étant imprimée sur ladite première couche isolante (34) selon un motif à mailles.

5. Système selon la revendication 1, ladite seconde section (23) présentant une longueur allant jusqu'à la moitié de ladite première longueur dudit connecteur ruban (10).

6. Système selon l'une quelconque des revendications précédentes, ladite première section (21) étant pleine.

7. Système selon l'une quelconque des revendications précédentes comprenant :
une pluralité de capteurs ;
chacune desdites bandes (25) se terminant par l'un de ladite pluralité de capteurs (14).

8. Système selon la revendication 7, ladite pluralité de bandes (25) dudit connecteur ruban (10) étant fixées de façon adhésive à ladite pluralité de capteurs (14).
